# EUROPEAN PATENT APPLICATION

(11) **EP 2 181 675 A1**
(43) Date of publication of application: **05.05.2010**
(21) Application number: 09425296.2
(22) Date of filing: 22.07.2009
(51) Int. Cl.: A61F 5/00, A61H 23/00

(54) **Machine to make the adjustment of the not in axis vertebrae by antalgica removal of paravertebral muscles**

(30) Priority: 18.09.2008 IT CL20080021
(71) Applicant: Pane, Emanuele, 93012 Gela (IT)
(72) Inventor: Pane, Emanuele, 93012 Gela (IT)

(57) **Abstract**

A machine designed to push the vertebrae by using an air cushion. The mechanical stimulus created by the work of the cushion, during the application, causes a heat sensation around the vertebrae and the roar of them. This system allows to gradually reaching the realignment of the backbone. This realignment allows getting recovery from some specific pathology such as: scoliosis, cervical osteoarthritis and every disease pertaining to the backbone malformation.

## Description

- Previous state of the tecnology: currently to treat specific pathologys such as scoliosis and others concerning to the backbone malformation, are used corsets, mechanical traction equipment, correttive gymnastics.

### Objective the inventions intends to reach

The capacity of the machine,object of the invention, is to cause a small and controlled push from the bottom to the top of the vertebrae , using as innovative criterion ,an air cushion. The mechanical stimulus , created by functioning of cushion during the applications, causes a heat sensation around the vertebrae (with the consequent relaxation of the paravertebral muscles) and the roar of them. This process allows to reach, with the necessary graduality, the realignment of the backbone with effective benefits on the mention pathologys.

The machine, realized as in illustration (FIG.A), structurally is composed by: -on air cushion inclusive three compartments separate each other, individually inflatable, since endow of singles inlet-(1),(2),(3) as in illustration ;
- A portable toolbox where are installed : two electrical pumps, two pressure regula= tors which have the function of regular, respectively, the flow and the downflow of air into the cushion, a digital timer, a manual switch and a flexible pipe for connecting the pumps to the cushion.

**OPERATING SYSTEM** : The cushion is putted under the patient's backbone, who is stretched out on a couch, on way that the three compartments A, B , C, are positioned transversely as regards of the length of the backbone.

Subsequently input the air into the compartment A which swell very slowly causes a little push from the bottom to the top. The same operation is repeated, in the same way, for the compartment B and C.

For obtain an order realignment of the vertebras, the passage from a comportment to another must be effected respecting the chronological order A,B,C and this independently by the position of the malformation ascertained along the patient's backbone. Carrying out this process is obtained, at the end of applications cycle, the antalgical removal of paravertebral muscles.

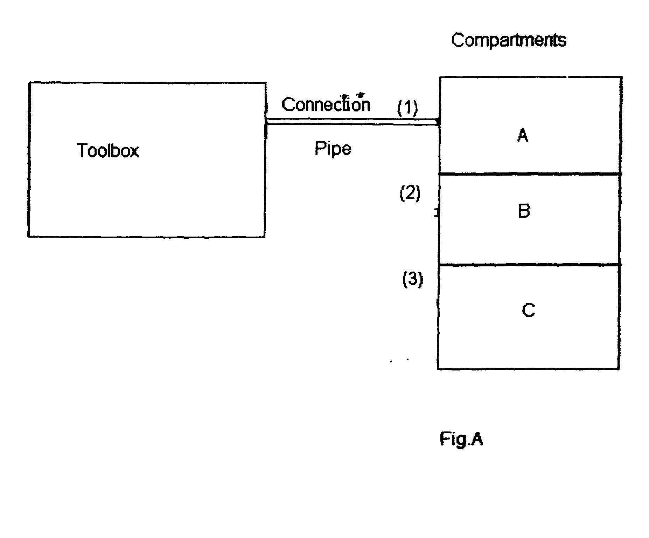

## Claims

1. The principle which machine is based on is the machanics push one provocated by tablet air flux which is admitted in an infeteble pillow , puts under the patient's shoulder and exactly where the back bon is, this air flux provokes a bit push(from the down to the top) of vertebrae not in board with the consequent antalgica removal of paravertebrali muscles.

2. The machine (which has been already realized) can be manufacture object, also industrial level, since it's composed by : a pillow with three partitions which can be inflated singly, a box where there are: two electrical pomps whith low potentiality, two regulating of pressure for regulate the flux and the down flow of air admitted on the pillow; a digital timer and a manual one; a flexible tube for connect the pumps to the pillow's air hold.
